# EUROPEAN PATENT SPECIFICATION

(11) **EP 4 289 396 B1**
(45) Date of publication and mention of the grant of the patent: **05.11.2025**
(21) Application number: 23173037.5
(22) Date of filing: 12.05.2023
(51) Int. Cl.: A61F 2/24

(54) **SHUNT TO AVOID VENTRICLE OVERLOAD AND CONDUIT FOR LEAD PLACEMENT**
SHUNT ZUR VERMEIDUNG VON VENTRIKELÜBERLASTUNG UND LEITUNG ZUR LEITUNGSPLATZIERUNG
SHUNT POUR ÉVITER LA SURCHARGE DU VENTRICULE ET CONDUIT POUR PLACEMENT DE DÉRIVATION

(30) Priority: 09.06.2022 US 202263350447 P
(43) Date of publication of application: 13.12.2023
(73) Proprietor: St. Jude Medical, Cardiology Division, Inc., St. Paul MN 55117 (US)
(72) Inventor: Von Oepen, Randolf, Aptos, CA (US); Licht, Grayston, Riverside, CA (US); Huddleston, Preston James, Maplewood, MN (US)
(74) Representative: LKGLOBAL Lorenz & Kopf Patentanwalt Attorney at Law PartG mbB

(56) References cited:
- WO-A1-2020/239686
- WO-A1-2021/080782
- US-A1- 2020 397 571
- US-A1- 2021 315 694

## Description

### Background of the Disclosure

The heart has four native valves, including the aortic valve, pulmonary valve, mitral valve (also known as the left atrioventricular valve), and the tricuspid valve (also known as the right atrioventricular valve). When these valves begin to fail, for example by not fully coapting and allowing retrograde blood flow (or regurgitation) across the valve, it may be desirable to repair or replace the valve. Prosthetic replacement heart valves may be surgically implanted via an open chest and open-heart procedure while the patient is on cardiopulmonary bypass. However, such procedures are extremely invasive, and frail patients, who may be the most likely to need a prosthetic heart valve, may not be likely to survive such a procedure. More recently, prosthetic heart valves have been trending to less invasive procedures, including collapsible and expandable heart valves that can be delivered through the vasculature in a transcatheter procedure.

Unless otherwise indicated, as used herein, the term "tricuspid valve" refers to the right atrioventricular valve, as opposed to just a generic term for a three-leaflet valve. Initial human trials to replace the native tricuspid valve in a transcatheter procedure (*e.g.,* via the femoral vein) have shown promising results, with patients experiencing significant improvements in quality of life after the prosthetic valve implantation. It is thought that important characteristics of a successful transcatheter prosthetic tricuspid valve device include not only a good clinical outcome for the patient, but the ease of use of the tricuspid valve, including for example having a small enough size to be able to avoid a surgical cut down of the patient's femoral vein for delivery.

WO 2021/080782 A1 describes devices, systems and methods providing treatment of a tricuspid valve. Such treatment may include tricuspid valve replacement, which may include providing a prosthetic tricuspid valve within the tricuspid valve annulus. Delivery systems for delivering the prosthetic tricuspid valve to the tricuspid valve annulus are described as well.

WO 2020/239686 A1 relates to a heart valve prosthesis with a variable sealing function, a method for replacing a native heart valve with a heart valve replacement prosthesis with a variable sealing function and the use of a heart valve replacement prosthesis with a variable sealing function for treating a heart valve disorder.

### Brief Summary of the Disclosure

The above-mentioned problems are solved or at least mitigated by the subject-matter of claim 1. Further embodiments are described by the dependent claims.

According to one aspect of the disclosure, a prosthetic heart valve system is for replacing a native right atrioventricular valve. The system includes a collapsible and expandable anchor frame. The anchor frame includes a central portion, and atrial and ventricular portions each flared radially outwardly from the central portion, the atrial and ventricular portions sized to sandwich or clamp an annulus of the right atrioventricular valve therebetween. An atrial sheet is coupled to the atrial portion of the anchor frame and extends radially inwardly to a central aperture in the atrial sheet, the atrial sheet being formed of a material that is substantially impermeable to blood. The system includes a generally cylindrical leaflet support structure, an inflow end of the leaflet support structure coupled to the atrial sheet. The atrial sheet defines an opening positioned radially outside of the leaflet support structure so that blood flowing around an exterior of the leaflet support structure can pass through the opening.

The leaflet support structure may be the central portion of the anchor frame, and an outer sealing fabric may extend between the atrial and ventricular portions of the anchor frame. In another embodiment, the anchor frame is an outer stent, and the leaflet support structure is an inner stent. In a further embodiment, the anchor frame is an outer stent, and the leaflet support structure is a fabric tube. The system may further include a collapsible and expandable prosthetic heart valve including a stent and a plurality of prosthetic leaflets, the prosthetic heart valve configured to be expanded into and received within the fabric tube of the anchor frame. In another embodiment, a plurality of prosthetic leaflets may be directly coupled to the leaflet support structure, the prosthetic leaflets forming a valve that allows blood to flow from the inflow end of the leaflet support structure to the outflow end of the leaflet support structure, but generally blocks blood from flowing from the outflow end of the leaflet support structure to the inflow end of the leaflet support structure. A radiopaque marker may be coupled to the atrial sheet adjacent to the opening.

The system further includes a ventricular sheet coupled to the ventricular portion of the anchor frame and extending radially inwardly to a central aperture in the ventricular sheet, wherein the ventricular sheet defines an opening positioned radially outside of the leaflet support structure, a tube extending from the opening in the ventricular sheet to the opening in the atrial sheet to form a shunt. The system may include an expandable occluder configured to be received within the shunt and to seal blood flow across the shunt, the occluder being formed as a braided mesh of metal strands. The system may also include an expandable stent configured to be received within the shunt and to limit blood flow across the shunt, the stent having an hourglass shape.

A closure member is coupled to the shunt, the closure member including a bioabsorbable member maintaining the closure member in an open condition in which blood is free to flow through the shunt. In one embodiment, the closure member is formed of a shape-memory material, the closure member including two apices and two "C"-shaped sides, the two "C"-shaped sides nesting with each other in the absence of applied forces. The bioabsorbable member may be a wire connected to the two apices of the closure member, the wire maintaining the closure member in an open condition in which the two "C"-shaped sides form a generally circular passageway, the tube of the shunt passing through the generally circular passageway. In another embodiment, the closure member is formed of a shape-memory material, the closure member having a closed condition in the absence of applied forces, the closure member forming a spiral shape with an interior diameter when in the closed condition. In the open condition of the closure member, two ends of the closure member overlap, the two ends being coupled together by the bioabsorbable member so that an interior diameter of the closure member in the open condition is larger than the interior diameter of the closure member in the closed condition. The bioabsorbable member may be a sheath that receives the two ends of the closure member within the sheath, or alternatively it may be a wire that wraps around the two ends.

The tube forming the shunt is either cylindrical or is bean-shaped in transverse cross-section. A semi-permeable membrane may be positioned within the shunt.

According to another aspect of the disclosure, not corresponding to the wording of the claims, a prosthetic heart valve system is for replacing a native right atrioventricular valve. The system may include a collapsible and expandable anchor frame. The anchor frame may include a support frame that has a central portion, and atrial and ventricular portions each flared radially outwardly from the central portion, the atrial and ventricular portions sized to sandwich or clamp an annulus of the right atrioventricular valve therebetween. The anchor frame may include an atrial sheet coupled to the atrial portion of the support frame and extending radially inwardly to a central aperture in the atrial sheet, and a ventricular sheet coupled to the ventricular portion of the support frame and extending radially inwardly to a central aperture in the ventricular sheet. The anchor frame may include a generally cylindrical leaflet support structure, an inflow end of the leaflet support structure coupled to the atrial sheet, and an outflow end of the leaflet support structure coupled to the ventricular sheet to provide a conduit from the central aperture in the atrial sheet to the central aperture in the ventricular sheet through the leaflet support structure. A first radiopaque marker may be positioned on the atrial sheet, and a second radiopaque marker may be positioned on the ventricular sheet. In the expanded condition of the support frame, a line of safe passage (*e.g.,* a line of clear or unobstructed) extends between the first radiopaque marker and the second radiopaque marker, the line of safe passage extending along a gap between the leaflet support structure and the central portion of the support frame.

According to a further aspect of the disclosure, not corresponding to the wording of the claims, a prosthetic heart valve system is for replacing a native right atrioventricular valve. The system may include a collapsible and expandable anchor frame. The anchor frame may include a support frame that has a central portion, and atrial and ventricular portions each flared radially outwardly from the central portion, the atrial and ventricular portions sized to sandwich or clamp an annulus of the right atrioventricular valve therebetween. The anchor frame may include an atrial sheet coupled to the atrial portion of the support structure and extending radially inwardly to a central aperture in the atrial sheet, and a ventricular sheet coupled to the ventricular portion of the support structure and extending radially inwardly to a central aperture in the ventricular sheet. The anchor frame may include a generally cylindrical leaflet support structure, an inflow end of the leaflet support structure coupled to the atrial sheet, and an outflow end of the leaflet support structure coupled to the ventricular sheet to provide a conduit from the central aperture in the atrial sheet to the central aperture in the ventricular sheet through the leaflet support structure. A guidewire may be pre-assembled to the prosthetic heart valve, such that in an expanded condition of the support frame, the guidewire extends through the atrial sheet and through the ventricular sheet along a pathway positioned radially outside of the leaflet support structure. The guidewire may have a first end extending beyond the atrial sheet and a second end extending beyond the ventricular sheet, an intermediate portion of the guidewire extending from the atrial sheet to the ventricular sheet. The system may include a catheter having a lumen sized and shaped to ride over the guidewire.

### Brief Description of the Drawings

Fig. 1 is a schematic illustration of the right atrioventricular valve.
Fig. 2 is a side view of an example of an outer or anchoring frame that may be used as part of a prosthetic tricuspid heart valve.
Fig. 3 is a cross-section of an anchor frame according to one aspect of the disclosure.
Fig. 4 is a top view of the anchor frame of Fig. 3.
Fig. 5 is a side view of a valve component that may be used with the anchor frame(s) of Figs. 2-4.
Fig. 6A is a cross-section of the anchor frame of Fig. 3 after occlusion of a shunt of the anchor frame.
Fig. 6B is a cross-section of the anchor frame of Fig. 3 after insertion of a secondary stent within a shunt of the anchor frame.
Fig. 7A is a side view of a shunt with closure members for self-closing the shunt.
Fig. 7B is a top view of the shunt of Fig. 7A with the closure members in an open condition.
Fig. 7C is a top view of the shunt of Fig. 7A with the closure members in a closed condition.
Fig. 8A is a top view of a closure member for use with a shunt according to another aspect of the disclosure.
Fig. 8B is a top view of the closure member of Fig. 8A maintained in an open condition by a first mechanism.
Fig. 8C is a top view of the closure member of Fig. 8A maintained in an open condition by a second mechanism different than the first mechanism of Fig. 8B.
Fig. 9 is a top view of the anchor frame of Fig. 3 with an alternate version of the shunt.
Fig. 10 is a cross-section of the anchor frame of Fig. 3 with an additional closing feature added to the shunt.
Fig. 11 is a cross-section of the anchor frame of Fig. 3, but instead of a shunt, the anchor frame includes radiopaque markers to assist placement of a guidewire in a similar location as the shunt of Fig. 3.
Fig. 12 is a cross-section of the anchor frame of Fig. 3, but instead of a shunt, with a pre-assembled guidewire in a similar location as the shunt of Fig. 3.
Fig. 13 is a cross-section of the anchor frame of Fig. 13 with a catheter positioned over the pre-assembled guidewire.
Fig. 14 is a cross-section of a single-stent prosthetic heart valve according to another aspect of the disclosure.
Figs. 15A-B are cross-section and perspective views, respectively, of a double-stent prosthetic heart valve according to another aspect of the disclosure.
Fig. 16 is a cross-section of the prosthetic heart valve of Fig. 15A with an alternate version of a shunt.

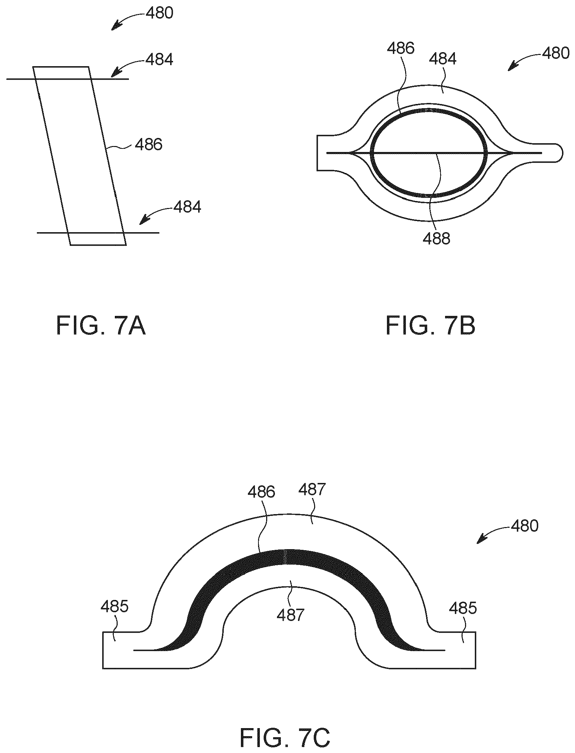

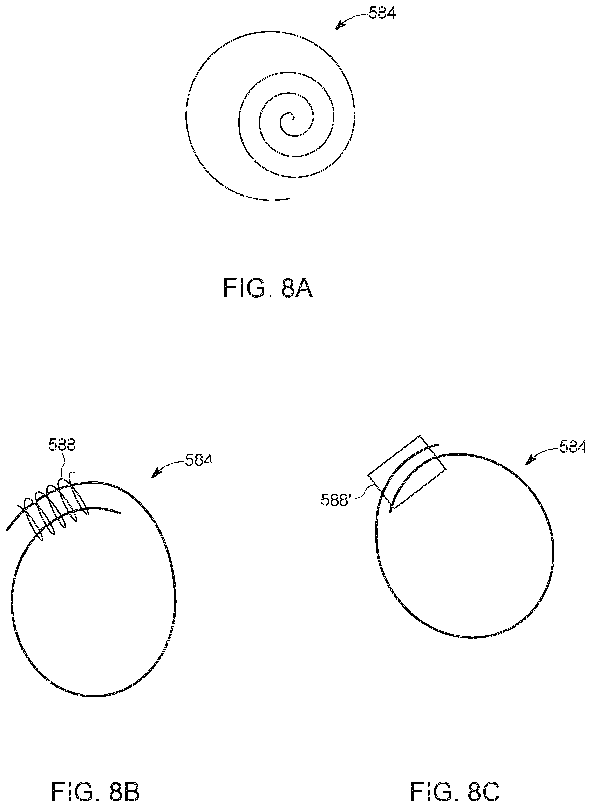

The invention relates to a prosthetic heart valve system with a closure member including a bioabsorbable member as illustrated in fig. 7, 8.

### Detailed Description of the Disclosure

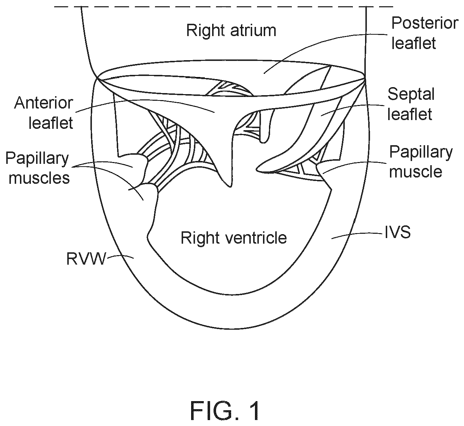

Fig. 1 is a schematic illustration of the right atrioventricular valve (commonly referred to as the tricuspid valve). The tricuspid valve separates the right atrium from the right ventricle, and typically includes three leaflets, which include a posterior leaflet, an anterior leaflet, and a septal leaflet. The septal leaflet is positioned nearest the interventricular septum ("IVS"). The tricuspid valve annulus may include conduction nodes near the connection point between the annulus and the septal leaflet, including for example the atrioventricular node ("AV node"). Electrical impulses may be conducted from the AV node, via the bundle of His, to the Purkinje fibers that provide electrical conduction to the ventricles. Papillary muscles along the right ventricular wall ("RVW") may support chordae tendineae coupled to the tricuspid valve leaflets to prevent inversion of the leaflets during normal physiological operation. The left atrioventricular valve (commonly referred to as the mitral valve) may have a generally similar structure as the tricuspid valve, though many differences do exist - including for example the mitral valve typically includes two leaflets (an anterior and posterior leaflet) and has the general shape of a hyperbolic paraboloid or "saddle"-type shape. Both the mitral valve annulus and tricuspid valve annulus may be very large compared to the aortic and pulmonary valves. For example, the tricuspid valve may have a diameter of between 45-50 mm in a patient with moderate tricuspid valve disease, and a diameter of between 50-60 mm in a patient with severe tricuspid valve disease. Diameters of up to 67 mm have been encountered in disease tricuspid valves, although even larger-sized annuluses may be encountered.

One important aspect of transcatheter tricuspid valve implantations is the size of components introduced into the patient's body. For example, it may be preferable for the delivery device that is used to deliver the transcatheter tricuspid valve to be small enough to be delivered through the femoral vein without requiring a surgical cut down of the vein. One desirable size example is about 30 French (10 mm) or below. Further, given the requirement for a transcatheter valve to be delivered through the patient's vasculature, the components of the system must be small enough to pass through the patient's vasculature safely. This size objective may be at odds with the objective of achieving strong anchoring, as more stent material may generally provide the ability for better anchoring, but also result in a larger device. In other words, the inclusion of a relatively large amount of anchoring structures in a prosthetic tricuspid valve may help provide better anchoring, but at a cost of increasing the overall size of the device (and thus the components, such as a delivery device, which will house the prosthetic heart valve).

Counterintuitively, it may also be preferable that a prosthetic tricuspid valve does not immediately resolve all tricuspid regurgitation. For example, in patients with severe or torrential tricuspid regurgitation, there may actually be a danger of transitioning between extreme tricuspid regurgitation to zero regurgitation in what is effectively an instantaneous change following implantation of a prosthetic tricuspid valve. This potential danger lies, at least in part, in the fact that the flow dynamics change nearly instantaneously, while the heart muscle cannot acclimate instantaneously. As an example, a patient with severe or torrential tricuspid regurgitation may have a relatively thin right ventricle due to dilation from heart failure. However, after tricuspid regurgitation is resolved, pressures within the right ventricle may increase, putting the patient at risk of right ventricular rupture or further dilation. This may place undue strain on the right ventricle. One solution to this issue, as described in greater detail below, is to include a shunt within the prosthetic tricuspid valve. The shunt, which may be temporary, may allow a certain amount of regurgitation from the right ventricle to the right atrium, even when the prosthetic leaflets of the prosthetic tricuspid valve are fully coapted. This shunt may reduce the pressure within the right ventricle, compared to what would be expected with zero regurgitation, but increase pressure within the right ventricle compared to the patient's disease state prior to the implantation. In other words, the combination of the coapting prosthetic leaflets and the shunt may decrease, but not eliminate, regurgitation. With this decrease in regurgitation, resistance is increased when the right ventricle contracts, which may allow the right ventricle to strengthen and/or thicken over a period of days, weeks, or months. During this period of days, weeks, or months, the shunt may be configured to naturally close to eliminate any remaining regurgitation, or otherwise may be actively closed during a secondary procedure. By the time the shunt closes, the right ventricle preferably has already acclimated to the new hemodynamics, so that upon complete (or substantially complete) elimination of regurgitation, the right ventricle has already gotten stronger and is more "ready" for the change in hemodynamics that occurs upon complete (or substantially complete) elimination of regurgitation.

Furthermore, in some patients, it may be advisable or necessary to implant a pacemaker along with (or shortly after) a prosthetic tricuspid valve implantation. Prosthetic heart valves may expand into contact with the AV Node, or otherwise cause interference with the natural conduction system of the heart. Thus, pacemakers may be implanted during, or after, a prosthetic heart valve implantation procedure to manage the patient's heart rhythm. If a shunt is included, the shunt may provide a passageway to assist with the placement of one or more pacemaker leads. And even if a shunt is not provided, other features are described below to assist in providing a passageway to assist with the placement of one or more pacemaker leads. Traditionally, pacemaker leads are passed through the center of the valve member of the prosthetic heart valve. In other words, pacemaker leads typically need to be delivered through an area where tissue leaflets (or in some cases fabric leaflets) are actively opening and closing as they perform their valve functionality. After implantation of the pacemaker leads, the leads may pass through the conduit formed by the prosthetic valve leaflets, but the pacemaker leads may create an obstruction that "pins" the prosthetic leaflets or otherwise inhibits the prosthetic leaflets from properly opening and/or closing. If the pacemaker leads inhibit proper coaptation of the prosthetic leaflets, regurgitation may occur. There is also a potential danger that the delivery device containing the pacemaker leads could damage the prosthetic leaflets if the delivery device is passed through the center of the prosthetic valve. Thus, the shunts described herein may provide for safe passageway and positioning of pacemaker leads such that the pacemaker leads (and/or a delivery device for same) do not need to pass through or ever be positioned within the area where the prosthetic leaflets are closing and opening. And as is described in greater detail below, even if a shunt is not included to provide a ready-made conduit for pacemaker implantation, other features may be provided to achieve a similar goal. Although general embodiments have been described above, more specific embodiments are disclosed below.

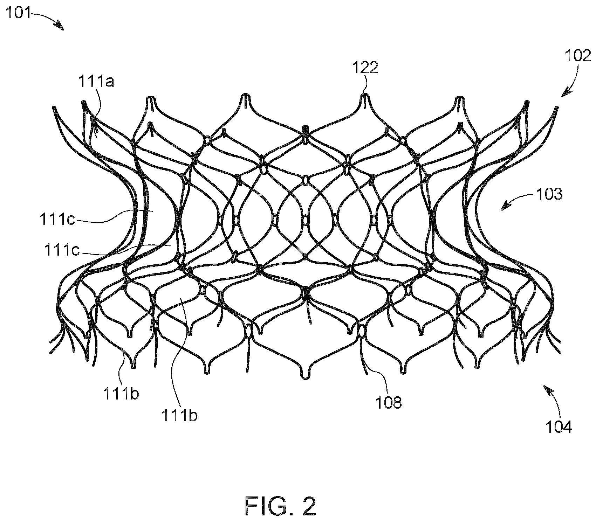

Fig. 2 is a side view of an exemplary anchoring or outer frame that may be used as part of the prosthetic tricuspid heart valves described herein. Anchor frame 101 is illustrated in an expanded condition in Fig. 2. The anchor frame 101 may be formed, in one example, via laser cutting a tube of shape memory material, such as a nickel-titanium alloy, including Nitinol, to form a stent or stent-like structure. After being formed, the anchor frame 101 may be shape-set to the expanded condition (*e.g*., by heat treatment), and the anchor frame 101 also has a collapsed condition in which the stent is collapsed to a smaller size from its expanded condition for transcatheter delivery to the patient. Anchor frame 101 includes an atrial portion or anchor 102, a ventricular portion or anchor 104, and a central portion 103 coupling the atrial portion to the ventricular portion. The central portion 103 is between atrial portion 102 and ventricular portion 104. Atrial portion 102 may be configured and adapted to be disposed on an atrial side of the tricuspid valve annulus and may flare radially outwardly from the central portion 103. Ventricular portion 104 may be configured and adapted to be disposed on a ventricle side of the native tricuspid valve annulus and may also flare radially outwardly from the central portion 103. This shape may help to self-center the anchor frame 101 upon deployment within the native tricuspid valve annulus, with the native tricuspid valve annulus being "sandwiched" or otherwise compressed between the atrial portion 102 and ventricular portion 104.

The atrial portion 102 may be formed as a portion of a stent or other support structure that includes or is formed by a plurality of generally diamond-shaped cells, although other suitable cell shapes, such as triangular, quadrilateral, or polygonal may be appropriate. In some examples, the atrial portion 102 may be formed as a braided mesh, as a portion of a unitary stent, or a combination thereof. According to one example, the stent that includes the atrial portion 102 may be laser cut from a tube of Nitinol and heat-treated to the desired shape so that the stent, including atrial portion 102, is collapsible for delivery, and re-expandable to the set shape during deployment. The atrial portion 102 may be heat treated into a suitable shape to conform to the native anatomy of the tricuspid valve annulus to help provide a seal and/or anchoring between the atrial portion 102 and the native tricuspid valve annulus.

The atrial portion 102 may include features for connecting the atrial portion to a delivery system. For example, the atrial portion 102 may include pins or tabs 122 around which sutures (or suture loops) of the delivery system may wrap so that while the suture loops are wrapped around the pins or tabs 122, the anchor frame 101 maintains a connection to the delivery device. However, in some embodiments, these pins or tabs 122 may be omitted.

The ventricular portion 104 may also be formed as a portion of a stent or other support structure that includes or is formed of a plurality of diamond-shaped cells, although other suitable cell shapes, such as triangular, quadrilateral, or polygonal may be appropriate. In some examples, the ventricular portion 104 may be formed as a braided mesh, as a portion of a unitary stent, or a combination thereof. According to one example, the stent that includes the ventricular portion 104 may be laser cut from a tube of Nitinol and heat-treated to the desired shape so that the ventricular portion 104 is collapsible for delivery, and re-expandable to the set shape during deployment. It should be understood that the atrial portion 102 and ventricular portion 104 may be formed as portions of a single support structure, such as a single stent or braided mesh. However, in other embodiments, the atrial portion 102 and ventricular portion 104 may be formed separately and coupled with one another. It should also be understood that the atrial portion 102 (which may be referred to as a disk) and the ventricular portion 104 (which may also be referred to as a disk) may each be substantially symmetric, or otherwise may each be asymmetric. As an example, there is only minimal annulus structure close to the atrial septum, and thus it may be preferable to have smaller stent cells and/or a smaller disk diameter in this region of the atrial disk, resulting in an asymmetric atrial disk. This may also mean that the anchoring stent 101 should be loaded in a certain orientation within the delivery device, or that the anchoring stent 101 can be rotated or will self-align so that the anchoring stent 101 is deployed in the desired rotational orientation relative to the patient's anatomy. And even if the atrial or ventricular disks are cut in a symmetric pattern, it might be desirable to heat set one or both structures so that the disks are asymmetric in their pre-set shape, for example curving more up or down compared to the rest of the structure. Also, it may be desirable that the cells in the atrial disk that are to be positioned closest to the atrial septum are softer to avoid any damage to the septal wall.

The anchor frame 101 may be configured to expand circumferentially (and radially) and foreshorten axially as the anchor frame 101 expands from the collapsed delivery configuration to the expanded deployed configuration. In the particular illustrated example, the anchor frame 101 includes or defines a plurality of atrial cells 111a in one circumferential row and a plurality of ventricular cells 111b in two circumferential rows. Each of the plurality of cells 111a, 111b may be configured to expand circumferentially and foreshorten axially upon expansion of the anchor frame 101. As shown, the cells 111a-b may each be diamond-shaped. In addition, a third plurality of cells 111c may be provided in additional circumferential rows, in this embodiment three additional center rows, forming the central portion 103.

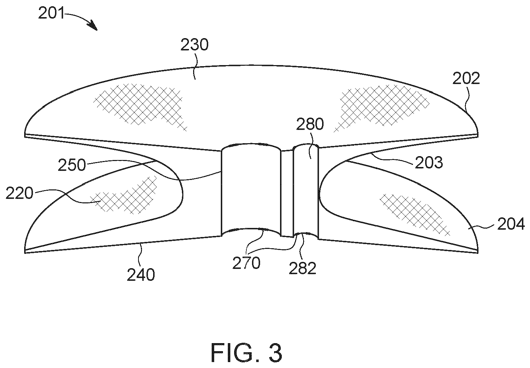

Still referring to Fig. 2, a pin or tab 122 may extend from an apex of each atrial cell 111a in a direction toward the outflow end of the anchor frame 101. Although one pin or tab 122 is illustrated in each atrial cell 111a, in other embodiments fewer than all of the atrial cells may include a pin or tab, and in some embodiments, the pins or tabs 122 may be completely omitted. In addition, each ventricular cell 111b may include a tine or barb 108 extending therefrom. In the illustrated embodiment, each tine extends from a point where two adjacent ventricular cells 111b in the terminal outflow row of cells meet one another, or otherwise from the outflow apex of a ventricular cell 111b in the second row of ventricular cells (*e.g.,* the row of ventricular cells directly adjacent, in the inflow direction, to the terminal outflow row of ventricular cells 111b). However, fewer than all of the ventricular cells 111b cells may include such tines or barbs. In the expanded condition of the anchor frame 101, as shown in Fig. 2, the barbs 108 may hook outwardly, the barbs being configured to pierce native tissue of the tricuspid valve annulus, such as the native leaflets, to help keep the prosthetic heart valve from migrating under pressure during beating of the heart. Typically, the term "tine" may refer to a structure configured to pierce into the tissue, while the term "barb" may refer to a tine that also includes a barb-like structure to prevent the barb from pulling out of the tissue once pierced. However, as used herein, the term "barb" includes tines, with or without actual "barb"-like structures that prevent pulling out of tissue, unless specifically noted otherwise. However, it should be understood that the tines 108 may be fully omitted from anchor frame 101 without a loss in the ability of the anchor frame 101 to suitably anchor the valve component without migration toward the right atrium. While tines or barbs have been used in prosthetic mitral valves to help resist atrial migration, the relatively low pressures experienced in the right heart may allow for the omission of such tines or barbs if desired. It should be understood that Fig. 2 only illustrates the metallic scaffold that forms the anchor frame 101 (which may be referred to as a stent), but other features including fabric skirts may be included, such as those shown and described in connection with Figs 3-4 below. Although the tines 108 are shown as being generally uniformly distributed around the ventricular disk, the tines 108 do not need to be uniformly distributed. For example, in order to avoid interference with the conductive system of the patient's heart, it may be desirable if, in the area of the AV node or the bundle of His, tines 108 are omitted, or the tines 108 in that location are formed with a different shape or structure to help avoid electrical interference with the conduction system of the heart.

Fig. 3 is a cross-section of an anchor frame 201 according to another aspect of the disclosure. As shown in Fig. 3, anchor frame 201 includes a flared atrial disk or atrial portion 202, a flared ventricular disk or ventricular portion 204, and a narrow-waisted central portion 203. The overall shape and structure of anchor frame 201 may be generally similar to that of anchor frame 101 and is thus not described again herein, other than to note that anchor frame 201 may be formed of a shape memory material such as Nitinol, and to note that the anchor frame 201 may be formed with a braided mesh or laser cut from a tube (*e.g.,* of Nitinol), for example with generally diamond-shaped cells that are shape-set to have the general hourglass shape shown in Fig. 3 in the absence of applied forces. In other words, anchor frame 101 and anchor frame 201 may be part of the same structure, but the illustration and description of anchor frame 101 focus on the stent or scaffolding component, while the illustration and description of anchor frame 201 focus on the other components that may be provided with the stent or scaffolding. As will become clear, anchor frames 101, 201 are not necessarily designed for direct coupling to an inner valve component, but rather to act as a first stage implant, to which a valve component is later secured. In this respect, anchor frames 101, 201 may be thought of as a "dock" for a prosthetic valve component. However, it should be understood that the shunts and shunt-related features described herein may apply to either a "dock" that is part of a two-stage prosthetic heart valve implant or to a single-stage prosthetic heart valve implant in which the prosthetic leaflets are pre-assembled to the support frame.

Anchor frame 201 may include one or more fabric components that may provide one or more functions, for example sealing. In the illustrated embodiment, the anchor frame 201 may include a sealing skirt 220 on a luminal and/or abluminal surface thereof. This sealing skirt 220 may be generally similar to other sealing skirts provided on stents or frames of transcatheter prosthetic heart valves. This luminal and/or abluminal sealing skirt 220 may be formed of any suitable material, including biomaterials such as bovine pericardium, or biocompatible polymers such as ultra-high molecular weight polyethylene, woven polyethylene terephthalate ("PET"), expanded polytetrafluoroethylene ("ePTFE"), or combinations thereof. The sealing skirt 220, particularly if positioned on the abluminal surface of the anchor frame 201, may include a "bump" (or gasket or ring) portion to enhance sealing, similar to that described in U.S. Patent Application No. 17/548,984. In addition to sealing skirt 220, the anchor frame 201 may include an atrial sheet 230 and a ventricular sheet 240. In some embodiments, any combination of sealing skirt 220, atrial sheet 230, and ventricular sheet 240 may be formed as an integral member, although in other embodiments, the atrial sheet 230 and ventricular sheet 240 are formed of different materials that provide different functionality and are not formed as integral members.

Referring still to Fig. 3, atrial sheet 230 may have a radially outward portion (*e.g.,* an outer circumferential area) coupled to the atrial portion 202, for example by suturing, and extend radially inwardly toward a central longitudinal axis where a central aperture is formed in the atrial sheet 230. But for the central aperture, the atrial sheet 230 may be thought of as a membrane not dissimilar to the head or skin of a drum. The ventricular sheet 240 may similarly have a radially outward portion (*e.g.,* an outer circumferential area) coupled to the ventricular portion 204, for example by suturing, and extend radially inwardly toward a central longitudinal axis where a central aperture is formed in the ventricular sheet 240. In other words, the atrial sheet 230 and ventricular sheet 240 may have substantially similar constructions, although they may be formed of different materials to provide different functionalities, preferably with the central apertures being substantially coaxial with each other when the anchoring frame 201 is in the expanded or deployed condition. The atrial sheet 230 and ventricular sheet 240 may be formed of any of the materials described above for sealing skirt 220, and/or any of the materials described below for the valve-receiving member 250.

A valve-receiving member 250, which may be generally cylindrical, may have a first inflow end coupled to the atrial sheet 230 so that the first inflow end is substantially coextensive with the central aperture of the atrial sheet 230, and a second outflow end coupled to the ventricular sheet 240 so that the second outflow end is substantially coextensive with the central aperture of the ventricular sheet 240. The valve-receiving member 250 is preferably formed of a fabric, such as PTFE, UHMWPE, Kevlar braid, Dacron, or biomaterials such as tissue. In some embodiments, the valve-receiving member 250 may be formed of thin wires of Nitinol, stainless steel, or other biocompatible metals or metal alloys formed into a braided, knitted, or woven structure. The inflow and outflow end of the valve-receiving member 250 may be coupled to the atrial sheet 230 and ventricular sheet 240, respectively, by any suitable means including sutures. At least in part because the valve-receiving member 250 is suspended within the anchor frame 201 via atrial sheet 230 and ventricular sheet 240, the valve component eventually received within the valve-receiving member 250 will retain its shape, even if the native tricuspid valve deforms the shape of the anchor frame 201, for example from forces as the heart contracts. In other words, the shape of the valve component within the valve-receiving member 250 is substantially independent of the shape of the anchor frame 201. This may be desirable because the valve component will typically have a circular shape or profile, and it is desirable to maintain that circular shape or profile to help ensure that the prosthetic leaflets of the valve component are able to coapt to prevent regurgitation across the prosthetic leaflets. If forces on the anchor frame 201 were transmitted to the valve component in the valve-receiving member 250, and the valve component was to be deformed, the prosthetic leaflets of the valve component may not be able to coapt correctly.

Although not required, it may be desirable to include radiopaque markers 270 on both the inflow end of the valve-receiving member 250 and the outflow end of the valve-receiving member 250. These radiopaque markers 270 may be formed from any biocompatible substance that is easily visualized during imaging and have any desired configuration. For example, the radiopaque markers 270 may be small pieces of biocompatible metal coupled to the valve-receiving member or radiopaque threads that are sutured into the valve-receiving member 250, although other configurations may be appropriate. These radiopaque markers 270 may readily show, during imaging, where the ends of the valve-receiving member 250 are located so that the valve component may be reliably and desirably positioned within the valve-receiving member 250 prior to expansion of the valve component. And, as described in greater detail below, the radiopaque markers 270 may also readily show the position of the shunt 280, if such a shunt is included. Examples of materials that may be used to form the radiopaque markers 270 may include materials with high atomic mass, such as gold, tungsten, platinum, and iridium as well as combinations or alloys of these materials. In some embodiments, it may be preferable to form the radiopaque markers 270 from a material that will enhance ingrowth into the material in order to more rapidly close shunt 280, a concept which is described in greater detail below.

Although the valve-receiving member 250 may be configured to receive a balloon-expandable or self-expandable prosthetic heart valve in a secondary procedure, in other embodiments the valve-receiving member 250 may include prosthetic leaflets directly coupled to the valve-receiving member 250. For example, bioprosthetic tissue (*e.g*., bovine or porcine pericardium) or synthetic fabric leaflets may be directly sutured to the interior of the valve-receiving member 250 so that the prosthetic heart valve may be implanted in a single step, instead of a two-step procedure. In embodiments in which the prosthetic leaflets are directly coupled to the valve-receiving member 250, it is preferable that the valve-receiving member 250 is formed of fabric or tissue to reduce the overall collapsed profile of the device, but in some embodiments, the valve-receiving member 250 may take the form of an inner metal stent that is collapsible and expandable. The shunt 280 described below may work equally well whether the prosthetic heart valve is designed as a two-stage or single-stage implant.

To expand on the point above, although the shunts are described herein along with illustrations and descriptions of a two-stage implant, the invention is not limited to two-stage implants. For example, the size of the inner valve of a prosthetic tricuspid valve may be generally between about 20 mm and about 36 mm in diameter (preferably between about 27 mm and about 33 mm), whereas the size of the native tricuspid valve annulus (in a patient with severe or torrential regurgitation) may be on average somewhere around 52 mm (*e.g*. between a range of about 36 mm to about 70 mm). Some single-stage prosthetic heart valves include two stents - an inner stent to support the prosthetic leaflets, and an outer stent for anchoring. In these embodiments, although the inner stent is coupled to the outer stent (*e.g.,* at an atrial side or ventricular side of the prosthetic heart valve), the size difference noted above results in a gap space being available between the outside of the inner stent and waist of the outer anchoring stent that is designed to contact the native valve annulus. The shunts described herein may be positioned within or along that gap space in substantially the same manner as described in connection with the exemplary two-stage implants shown herein.

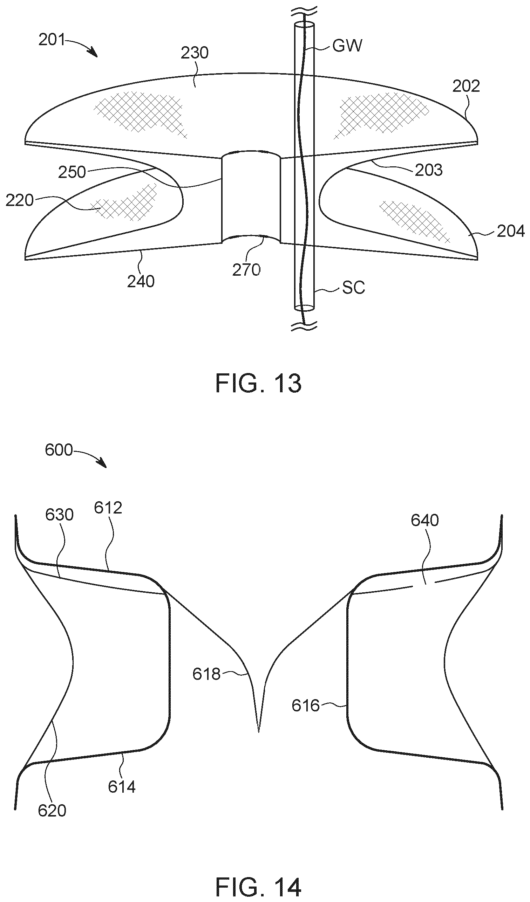

To expand on the point above even further, some recently developed prosthetic tricuspid heart valves include a single stent that has a relatively small (*e.g*., between about 27 mm and about 33 mm) central diameter to directly house the prosthetic leaflets, and relatively large (*e.g.,* 50 mm or more) atrial and ventricular disks. Examples of those recently developed prosthetic tricuspid heart valves are described in greater detail in U.S. Patent Application No. 63/341,702 filed May 13, 2022. Those recently developed prosthetic tricuspid valves include a fabric covering extending between the large atrial and ventricular disks, and it is that fabric covering that directly presses against the native tricuspid valve annulus. In these recently developed prosthetic valves, there is similarly a gap space between the outside of the center portion of the stent that houses the prosthetic leaflets, and the fabric that presses against the native tricuspid valve annulus upon implantation. That gap space may be used either to place a shunt, or to otherwise allow for shunt-like activity, as described in greater detail in connection with Fig. 14.

The shunt 280 is a tube that provides a pathway for blood to flow in an unrestricted fashion (at least initially). The shunt has a first open end coupled to the atrial sheet 230, and a second open end coupled to the ventricular sheet 240. Preferably, the material forming the tube of the shunt 280 is substantially impermeable to blood, so that blood only flows between the two ends of the shunt 280. For example, when the right atrium contracts, blood may flow through both the shunt 280 and through the open leaflets positioned within valve-receiving member 250. When the right ventricle contracts, the leaflets positioned within the valve-receiving member 250 completely (or substantially completely) block blood from flowing in the retrograde direction through the valve-receiving member 250, but the shunt 280 allows for the unimpeded flow of blood in the retrograde direction through a flow opening 282 of the shunt 280, limited mainly by the size of the lumen of the shunt 280. In some examples, the shunt 280 may have a diameter of between about 2 mm and about 10 mm. In other examples, the shunt 280 may have a diameter of between about 4 mm and about 8 mm. However, it should be understood that these diameters are merely exemplary. Prior to the shunt 280 being closed, whether in a secondary procedure or as the result of the shunt 280 being designed to self-close over time, the shunt 280 may also be used as a lumen through which a pacemaker lead may be placed during a pacemaker implantation procedure. And although the shunt 280 is described as being substantially impermeable to blood, in some embodiments, the shunt 280 may be formed of a material that is permeable to blood. For example, as is described in greater detail below, it may be preferable to form the shunt 280 from a material that promotes ingrowth so that the shunt 280 seals over time. In these embodiments, it may be desirable to form the shunt 280 from a material that is porous and/or permeable to blood as such materials may enhance ingrowth.

In one embodiment, the shunt 280 may be formed as a substantially cylindrical tube of fabric, which may be formed of any of the materials described in connection with atrial sheet 230 or ventricular sheet 240. The atrial sheet 230 and ventricular sheet 240 may include openings where the respective ends of the shunt 280 are coupled to the two sheets, in a similar way as described in relation to the connection of the valve-receiving member 250 to the atrial sheet 230 and ventricular sheet 240. Also, one or both ends of the shunt 280 may include radiopaque markers 270, in a similar or identical fashion as described in connection with valve-receiving member 250. The main body of the shunt 280 may be positioned radially outside of the valve-receiving member 250, and radially inside the central portion 203 of the stent of the anchor frame 201. In other embodiments, described in greater detail, the shunt is instead positioned radially outside of an inner stent, and radially inside either an outer anchoring stent, or radially inside an outer fabric that directly contacts the native valve annulus.

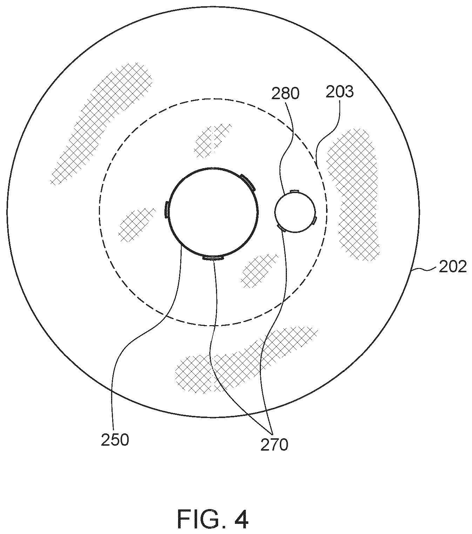

Fig. 4 is a top view (of the atrial side) of the anchor frame 201 when in the expanded condition. As shown, the valve-receiving portion 250 results in an open passageway axially through the anchor frame 201, while the shunt 280 also results in an open passageway axially through the anchor frame 201, but of a smaller diameter than the valve-receiving member 250.

In one exemplary use of anchoring frame 201, it may first be transitioned to a collapsed condition and placed within a sheath of a delivery device, the sheath maintaining the anchoring frame in the collapsed condition. The sheath of the delivery device may have an outer diameter of up to about 38 French (12.67 mm), although it is preferably smaller and has a diameter of between about 30 French (10 mm) and 38 French (12.67 mm), and it is most preferably even smaller with a diameter of between about 20 French (6.67 mm) and about 28 French (about 9.33mm). After the anchoring frame 201 is within the delivery device, it may be introduced into the femoral vein. If the device is small enough (*e.g.* 33 French (11 mm) or smaller, preferably 30 French (10 mm) or smaller), it may be introduced into the femoral vein without the need for a surgical cut down of the femoral vein. The delivery device may be advanced through the patient's vasculature, through the inferior vena cava, into the right atrium, and may be oriented (for example via a steering mechanism) so that the distal end of the sheath is within or adjacent to the native tricuspid valve annulus. The distal end of the sheath may be withdrawn relative to the anchoring frame 201, removing the constraint on the anchoring frame 201 and allowing the anchoring frame 201 to begin to self-expand into the native tricuspid valve annulus, with the ventricular portion 204 abutting the ventricular side of the valve annulus, the atrial portion 202 abutting the atrial side of the valve annulus, and the valve annulus received within the waisted central portion 203. When deployed, the anchor frame 201 may provide reliable anchoring via (i) the pinching of the native tricuspid valve annulus by the atrial portion 202 and the ventricular portion 204; (ii) the oversizing of the central waist portion 203 relative to the native tricuspid valve annulus; and/or (iii) anchoring tines or barbs, if included. However, it should be understood that the anchor frame 201 may be designed so that oversizing of the central waist portion 203 relative to the native annulus is not needed. In fact, although the three above-described modalities of anchoring may be provided in a single device, any combination of anchoring mechanisms (i), (ii), and/or (iii) listed above may be sufficient. In some embodiments, the oversizing of the ventricular portion 204 relative to the native valve annulus may help the valve remain in place by providing a mechanism for resisting migration into the right atrium. While the anchor frame 201 is deployed, and before the valve component is deployed into the valve-receiving member 250 (which may be referred to as the first stage of implantation), the valve-receiving member 250 provides an open conduit between the right ventricle and the right atrium. If the anchor frame 201 is designed as a single-stage implant with prosthetic leaflets already attached to the valve-receiving member 250, the initial valve implantation may be complete at this point. If the anchor frame 201 is designed as a two-stage implant, the valve prosthesis may be implanted next as outlined below.

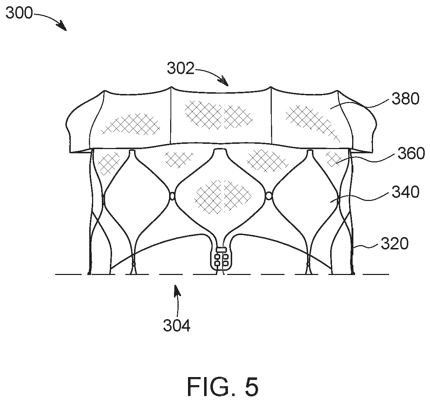

Once the first stage of implantation has been completed and the anchor frame 201 is deployed within the native tricuspid valve annulus, the valve-receiving member 250 creates an open conduit between the right atrium and the right ventricle. Preferably, there is little or no delay between completing the first stage of implantation and beginning the second stage of implantation. In the second stage of implantation (if required), a valve component 300 is deployed into the anchor frame 201. Fig. 5 illustrates an exemplary valve component 300, but it should be understood that other configurations of valve components may be utilized in a similar or identical fashion. Referring to Fig. 5, a valve component 300 is illustrated that is similar to the Navitor^{™} prosthetic heart valve offered by Abbott Labs. One main difference of the valve component 300 shown in Fig. 5 compared to the Navitor^{™} device is that valve component 300 does not include an outward stent flare at the outflow end 304 of the device. However, the portion of the valve component 300 housing the prosthetic leaflets 340 may be substantially similar or identical to the Navitor^{™} device. Generally speaking, valve component 300 extends from an inflow end 302 to an outflow end 304 and includes a stent 320, a plurality of prosthetic leaflets 340 (in this embodiment, three prosthetic leaflets 340, preferably formed of pericardial tissue or synthetic biocompatible materials), an inner skirt or cuff 360 on a luminal surface of the stent 320, and an outer skirt or cuff 380 on an abluminal surface of the stent 320. In this particular example, stent 320 has a cylindrical annulus portion nearer the inflow end 302 and excludes any outwardly flared outflow portion nearer the outflow end 304. Further, in this particular example, stent 320 is formed of a plastically expandable material, such as stainless steel, cobalt-chromium, or other metals or metal alloys that are plastically expandable, so that the valve component 300 is balloon expandable. The illustrated cylindrical profile of valve component 300 may be particularly suited for a balloon-expandable valve. However, in other embodiments, the stent 320 may have other shapes, including a radially outward flare (*e.g*., similar to, or less pronounced than, what is provided in the Navitor^{™} device). Still further, in other embodiments, the stent 320 may be formed of a shape memory material, such as nickel titanium alloy, such as Nitinol, so that the valve component 300 is self-expandable. If the valve component 300 is self-expandable, it may be particularly useful to include the outward flare at the outflow end of the valve component 300, as such an outward flare may help with anchoring, whereas balloon-expandable valves may be anchored via forces from balloon expansion.

The prosthetic leaflets 340 may have, in the aggregate, a generally cylindrical profile, with three leaflets total, each leaflet coupled to an adjacent leaflet at a commissure feature of the stent 320. However, more or fewer prosthetic leaflets 340 may be provided as desired. The inner cuff 360 may be formed of biocompatible tissue or synthetic material, such as PTFE, PET, or ultra-high molecular weight polyethylene (UHMWPE). The outer cuff 380 may similarly be formed of biocompatible tissue or synthetic material, such as PTFE, PET, or UHMWPE. In the illustrated configuration, the outer cuff 380 has an inflow edge that is fixed (*e.g.,* via suturing) to the inner cuff 360 and/or the stent 320, with an outflow edge that is coupled to the inner cuff 360 and/or stent 320 at spaced apart circumferential locations, to create one or more openings between the outer cuff 380 and the inner cuff 360 into which blood may flow. If blood flows into these openings, *e.g*., during retrograde blood flow, the outer cuff 380 may billow outwardly to help ensure there is no regurgitation around the outside of the valve component 300. Additional details that may be relevant for use with valve component 300 are described in greater detail in U.S. Patent No. 10,548,722. However, because valve component 300 is, in this embodiment, intended to be received within valve-receiving member 250, it should be understood, that the inner cuff 360 and/or outer cuff 380 may be omitted, with the valve-receiving member 250 itself providing cuff/skirt functionality.

The valve component 300 may be collapsed to a small diameter and positioned within the sheath of a delivery device and introduced into the right heart via any suitable means and delivery route. For example, the valve component 300 may be delivered via the femoral vein, similar to anchor frame 201, via a transapical access route through the chest and through the right ventricle, via a transjugular delivery route and through the superior vena cava, or any other desirable delivery route that leads to the tricuspid valve. In some circumstances, particularly if the second stage implantation is being performed immediately after the first stage, the same catheter may even be used for each stage of implantation, although this is not required.

Regardless of the delivery route, the distal end of the delivery catheter housing the collapsed valve component 300 is positioned adjacent to, or within, the valve-receiving member 250 of the anchor frame 201.

If valve component 300 is self-expandable, the catheter sheath may be withdrawn or advanced to uncover the valve component 300, removing the constriction maintaining the valve component 300 in the collapsed condition, thus allowing the valve component 300 to self-expand into the valve-receiving member 250. If the stent 320 of valve component 300 includes an outwardly flared outflow section, the outward flare may protrude beyond the outflow end of the valve-receiving member 250 and provide additional anchoring force to resist migration toward the right atrium. However, in other embodiments, the outwardly flared outflow section may be omitted, with the stent being generally cylindrical.

If valve component 300 is balloon-expandable, the valve component 300 may be crimped over an uninflated balloon when the valve component 300 is mounted to the delivery catheter in the collapsed condition. However, if the valve component 300 is balloon-expandable it may or may not be covered by a catheter sheath during the second stage implant. Rather than withdrawing a sheath to allow the valve component 300 to self-expand, the balloon is inflated (*e.g*., by pushing saline through the delivery device into the balloon) to force the valve component 300 to expand into the valve-receiving member 250 of the anchor frame 201. Regardless of whether the valve component 300 is self-expanding or balloon-expandable, the radiopaque markers 270 on the inflow and/or outflow end of the valve-receiving member 250 (if included) may be referenced with imaging to confirm that the valve component 300 is in the desired position relative to the valve-receiving member 250 prior to expansion of the valve component 300.

Whether self-expanding or balloon-expandable, it is preferable that the valve component has a diameter (at least at the portion housing the prosthetic leaflets 340) of between about 25mm and about 35mm, including between about 28mm to about 32mm, including about 29mm, about 30mm, and about 31mm.

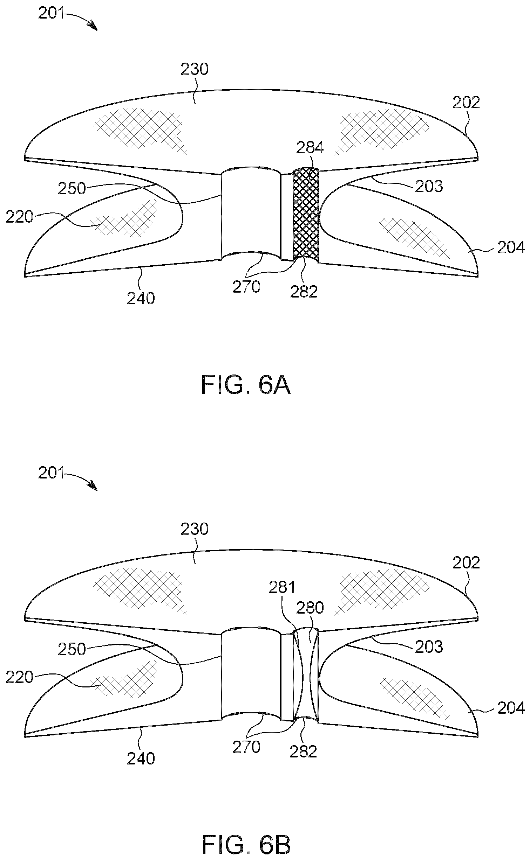

After the valve component 300 is deployed within the anchor frame 201, the prosthetic leaflets 340 may take on the function of the previously failing native tricuspid valve leaflets. At this point, any remaining catheters or other accessories within the patient may be removed, and the procedure completed. As noted above, as the patient recovers from the prosthetic heart valve implantation, the intentional regurgitation of blood across the shunt 280 may allow the heart to acclimate to the new hemodynamics without overloading the right ventricle. Thus, over time, the patient's right ventricle may become stronger and, after the patient has recovered from the prosthetic heart valve implantation, the shunt 280 may be closed in a separate procedure. For example, an occluder may be implanted into the shunt 280 to occlude the passageway, eliminating any regurgitation of blood across the shunt 280. The occluder may take any suitable form that is configured to be positioned within and/or through the shunt 280 and to either immediately or over time occlude the shunt 280 so that blood can no longer flow through the shunt 280. In one embodiment, as shown in Fig. 6A, an occluder 284 may take the form of a metal mesh, which may be for example formed as a braided mesh of strands of metal. However, it should be understood that occluders may have various other designs, including meshes made from materials other than metal braids, and even other non-mesh configurations. In one embodiment, the strands may be formed of Nitinol and the occluder 284 may be formed as an expandable occluder. In the illustrated embodiment, occluder 284 is a generally cylindrical member that can be collapsed down to a small size for transcatheter delivery, with the catheter carrying the occluder 284 passing through the shunt 280 during delivery, for example with guidance based on the radiopaque markers 270. Once the catheter is positioned within the shunt 280, the occluder 284 may be deployed from the catheter, causing the occluder 284 to expand into frictional engagement with the shunt 280. The occluder 284 may include one or more fabrics within the interior of the occluder 284 to further help create a seal across the shunt 280. In some embodiments, one or both ends of the occluder 284 may be flared with the flare extending beyond the end of the shunt 280 to provide enhanced anchoring. Other occluder designs may be suitable for use as the occluder 284, including devices already commercially available such as the Amplatzer^{™} line of occluders offered by Abbott Labs, such as the Amplatzer^{™} Duct Occluder. It should be understood that other occluder designs may be suitable, and any occluder capable of minimally invasive delivery, creating an effective seal across the shunt 280, and anchoring within the shunt 280 may be suitable for use. With the shunt 280 closed, regurgitation across the prosthetic tricuspid valve will be eliminated (or substantially eliminated), but the heart, and particularly the right ventricle, will have had time to acclimate to the new pressure dynamics and the risk of right ventricle overload may have substantially decreased by allowing the shunt 280 to remain open for a time after the initial procedure.

As shown in Fig. 6B, instead of fully closing the shunt 280 with an occluder 284, in other embodiments, an expandable stent 281 having an hourglass shape (*e.g*., wider terminal ends with a waisted central portion) may be implanted into the shunt 280 during a secondary procedure. The stent 281 may be covered with a blood-impermeable fabric. After the stent 281 expands (*e.g*., via self-expansion or balloon expansion using an hourglass-shaped balloon), the area through which blood may flow through the shunt 280 reduces to the narrow area of the central waist of the stent 281. With this configuration, the shunt 280 is not occluded via implantation of the stent 281, but rather the volume of regurgitation through the shunt 280 is reduced. In this manner, during the secondary procedure, the amount of regurgitation through the shunt 280 may be reduced and fine-tuned to the desired amount. If such as stent 281 is used in a secondary procedure, it may include features to fully occlude over time, including any of the mechanisms described below.

In the embodiment described above, the shunt 280 is closed as part of a separate, second procedure. In other embodiments, the shunt 280 may be designed so that a separate procedure is not necessary to close the shunt 280, but rather the shunt 280 will naturally close over time by virtue of its design.

One example of a self-closing shunt 480 is illustrated in Figs. 7A-C. Fig. 7A is a side view of the shunt 480 isolated from other components of the prosthetic heart valve system. Shunt 480 may be pre-assembled to the prosthetic heart valve in substantially the same fashion as shunt 280, with the only difference being that shunt 480 includes a closure mechanism. As with shunt 280, shunt 480 may include a tube 486 that is open at each end, with the fabric being either permeable or substantially impermeable to blood across the fabric. The material forming tube 486 (or the tube of any other shunt disclosed herein) may be formed of any suitable biocompatible material, such as a knitted or woven PET, PTFE, or similar material, or a tissue material such as bovine or porcine pericardium. Although not shown, the tube 486 may be coupled to (*e.g.,* via suturing) atrial and ventricular sheets of an anchor frame in substantially the same manner as described above for shunt 280. Shunt 480 is illustrated with two substantially identical closure members 484, one near each end of the tube 486. However, it should be understood that a single closure member 484, or more than two closure members 484, may be used and the positioning of the closure members 484 may be different than that shown in connection with Fig. 7A. One closure member 484 is shown from a top view in Fig. 7B in an open condition. The closure member is preferably formed of a shape-memory alloy such as Nitinol. The closure member 484 has an open condition, as shown in Fig. 7B, and a closed condition, as shown in n Fig. 7C. Generally, the closure member 484 may be formed as a diamond-shaped member or two opposing "C"-shaped members which are joined together to form opposite apices. In one example, each closure member 484 is formed of a continuous piece of Nitinol that has two apices 485 and two edge members 487 extending between the two apices 485. The closure member 484 may be shape-set, for example by heat treatment, so that in the absence of applied forces, the closure member 484 has the configuration shown in Fig. 7C in which the two edge members 487 closely nest against each other. For example, if each edge member 487 has a "C"-shape, in the absence of applied forces, the convex curvature of one edge member 487 nests or presses against the concave curvature of the other edge member 487. Although two "C"-shapes are shown, other shapes may be suitable to achieve the same goal, including diamond shapes or other shapes that can be held open by a biodegradable suture that is under tension. As one specific example of an alternative closed shape, the two edge members 487 may each move inwardly toward each other (*e.g.* as the two apices 485 move away from each other) so that the two edge members 487 become substantially parallel and are positioned close to each other, which may or may not include contacting each other. With this alternative closed shape, the two edge members 487 generally form two parallel bars that close the shunt 480. And while Nitinol is described as one material option for the closure member 484, it should be understood that other materials, including spring steel or plastics, may also work well for this feature, particularly as the closing forces do not need to be large for the closure member 484 to be effective.

Referring to Fig. 7B, the closure member 484 may be maintained in the open condition via a wire 488 which preferably is a suture or other string-like member that is biodegradable or bioabsorbable. Although the term "wire" is used, it should be understood that the term "wire" itself does not require any particular material property and is merely a generic term that encompasses string-like and suture-like structures. The wire 488 may have two ends, each end being coupled to one of the apices 485 of the closure member 484. The wire 488 may be attached to the apices 485 while the closure member 484 is maintained in the open condition, and following attachment, the wire 488 may be in tension, forcing the closure member 484 to stay in the open condition shown in Fig. 7B as long as that tension is maintained. The tube 486 of the shunt 480 may pass through the open center of the closure member 484, and the tube 486 may be coupled to the closure member 484, for example via suturing along the side edges 487 and/or the apices 485. Following implantation, the exposure of the bioabsorbable wire 488 to the flow of blood will tend to slowly break down the wire 488. Eventually, the wire 488 will break down enough so that the wire 488 breaks at some point along its length, causing the wire 488 to lose tension and thus causing the wire 488 to stop applying force to the closure member 484 that maintains the closure member 484 in the open condition. After the applied force from the wire 488 is removed, the closure member 484 will tend to change back to the shape set, shown in Fig. 7C. Because the edge members 487 of the closure member 484 nest or press against each other after returning to the set shape, the two edge members 487 effectively clamp or pinch the tube 486 so that blood can no longer flow through the tube 486. At this point, the shunt 480 is closed and blood can no longer regurgitate through the shunt 480. In other words, the result of the occlusion of shunt 280 is achieved without the need for an additional procedure since shunt 480 is capable of self-closing with time. While a single closure member 484 may be effective to close the shunt 480, the use of two or more closure members 484 may enhance the ability to achieve a complete seal across the shunt 480. Although wire 488 is described as being bioresorbable, it should be understood that, in other embodiments not covered by the invention, the wire 488 may not be bioresorbable. In such embodiments, the wire 488 would be durable and allow for the physician to cut the wire, in a later procedure, at the exact timing preferred by the physician. Although this may require an extra interventional step, it may also provide the physician with more control regarding the timing of the shunt closure.

Fig. 8A illustrates a variation of a closure member 584 that may be used with shunt 480 in a similar fashion as described for closure member 484. Fig. 8A illustrates the closure member 584 in a closed condition. The closure member 584 may be formed from a wire of shape-memory material, such as Nitinol. The closure member 584 may be shape set, for example by heat treatment, to the closed condition shown in Fig. 8A in which the closure member 584 forms a spiral configuration or otherwise includes a plurality of substantially concentric loops of decreasing diameter. When assembling the shunt, the closure member 584 may be stretched open to form a substantially circular shape, as shown in Figs. 8B-C, with opposite ends of the wire forming the closure member 584 having no overlap or only a slight overlap. In the embodiment of Fig. 8B, a biodegradable or bioabsorbable wire 588 wraps or winds around the overlapping free edges of the closure member 584 to apply a force that maintains the closure member 584 in the open condition. In the embodiment of Fig. 8C, the two terminal ends of the closure member 584 are positioned within and bound together by a biodegradable or bioabsorbable sheath 588'. Again, the sheath 588' applies a force that maintains the closure member 584 in the open condition. Although not shown, a plurality of closure members 584 may be provided and a fabric tube similar to fabric tube 486 may extend through the center of each closure member 584, and the closure members 584 may be coupled to the tube of the shunt for example by suturing. Closure member 584, whether maintained in the open position by a winding wire 588 or tubular sheath 588', operates similarly to closure member 484. Upon implantation, the closure member 584 is forced to remain in the open position, keeping the fabric tube of the shunt open for free regurgitation across the shunt. Over time, as contact with blood causes the winding wire 588 or tubular sheath 588' to biodegrade or bioresorb, the forces applied on the closure member 584 reduce. As these forces reduce, the closure member will tend to revert to its set shape as shown in Fig. 8A, either in a rapid fashion after only a threshold applied force remains, or in a more gradual fashion with the closure member gradually coiling upon itself. In the closed condition of Fig. 8A, the fabric tube within the closure member 584 is clamped or pinched to block blood from flowing through the shunt.

Although various materials may be suitable for forming the biodegradable or bioabsorbable wires or sutures described above, typically these sutures are made from Polylactide acid, Polyglycolide acid, or copolymers of these materials. These biodegradable sutures may be designed to have any desired range of degradation time, with the degradation time being influenced by factors including the makeup of the material forming the sutures, as well as the molecular weight of the material.

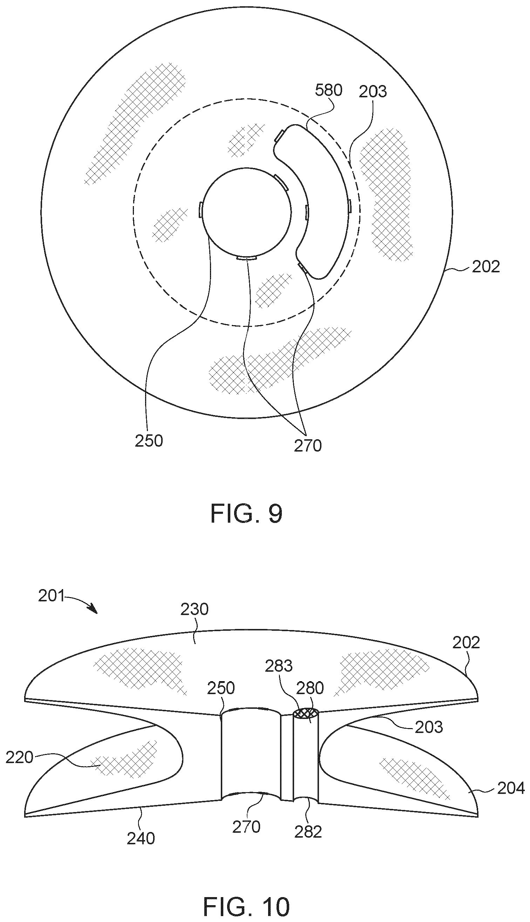

Fig. 9 is a top view (of the atrial side) of the anchor frame 201 of Figs. 3-4, with shunt 280 being instead replaced with shunt 580. Other than the shape of the shunt 580, and the resulting positions of the radiopaque markers 270, the anchor frame of Fig. 9 is identical to that of Figs. 3-4. Whereas shunt 280 is formed in the shape of a cylinder (*e.g.,* a right cylinder) with a transverse cross-section having a circular shape, shunt 580 is formed as having a bean shape in cross-section. In other words, the transverse cross-section of shunt 580 may have a radially outer curve having a relatively large radius of curvature, and a radially inner curve having a relatively small radius of curvature, with the inner and outer curves being connected to form rounded ends. In the illustrated embodiment, radiopaque markers 270 are positioned near the center of the inner and outer curves, as well as the curved portions connecting the inner and outer curves. This is just one example of a shape that may be suitable for shunt 580 that is not a right cylinder, and other shapes may be appropriate. The shape may be chosen, at least in part, based on the desired flow through the shunt and the ease with which the shunt may be closed later, whether via a separate occlusion procedure or a self-closing process.

As described above, the various shunts described herein may be closed via a secondary procedure or intervention, or otherwise may be self-closing. Instead of mechanical action to close the shunts, as described in connection with Figs. 7A-8C, the shunts may be designed to close via ingrowth after implantation. For example, Fig. 10 shows the anchor frame 201 of Fig. 3 with identical features as those shown in Fig. 3, and one additional feature. The only difference between the anchor frame 201 of Figs. 3 and 10 is that the anchor frame 201 of Fig. 10 includes a semi-permeable membrane or cover 283 that covers the inflow end of the shunt 280. The semi-permeable cover 283 may be formed as fibers, fabric, or other materials that promote cell growth. In some examples, the semi-permeable cover 283 may be formed of UHMWPE, woven PET, ePTFE, or combinations thereof. Although the semi-permeable cover 283 is shown on the inflow end of the shunt 280, it may instead be provided on the outflow opening 282 of the shunt 280, or on both ends of the shunt 280. Immediately after implantation of the prosthetic heart valve, blood will be able to flow through the shunt 280 and pass through the semi-permeable cover 283 that extends across the lumen of the shunt 280. As time passes, however, cell growth into the semi-permeable cover 283 will cover, and as additional cell growth occurs, the semi-permeable cover 283 becomes less porous and eventually, the ingrowth will cause the cover 283 to block blood from flowing across the cover. Once the cover 283 is blocking blood from flowing, the shunt 280 is effectively closed. In some embodiments, and as noted above, the tube forming the shunt 280 may itself also be formed of a semi-permeable material to enhance the ingrowth to occlude the shunt 280 over time.

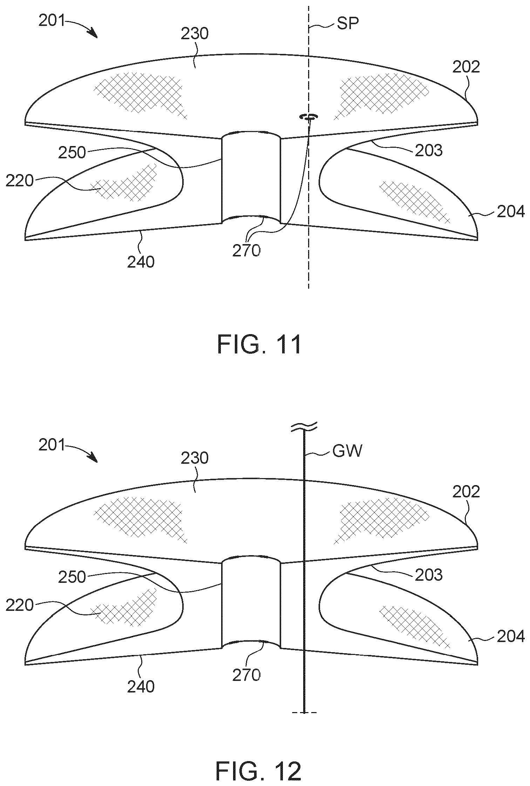

In all of the embodiments described above, the shunts are pre-assembled to the prosthetic heart valve so that the shunts are functioning immediately upon implantation of the anchor frame, allowing for regurgitation through the shunt as soon as the anchor frame is implanted. However, in some embodiments, it may be desirable to exclude such a shunt. For example, if a patient does not appear to be at risk of right ventricular overload, the inclusion of a shunt may not be desirable. However, those patients still may require a pacemaker to be implanted after the prosthetic heart valve is implanted. In the embodiments with shunts described above, the shunts may act as a convenient delivery pathway that the pacemaker leads may be passed through during implantation, prior to closure of the shunt, while eliminating the risk that the delivery of the leads will damage the prosthetic leaflets and the risk that the lead placement may interfere with proper coaptation of the prosthetic leaflets. However, if no shunt is included, features may be provided to assist with pacemaker implantation. For example, Fig. 11 illustrates a cross-section of anchor frame 201 that is identical to that shown in Fig. 3, with only two exceptions. The first exception is that no shunt is pre-assembled to the prosthetic valve, and as a result, the atrial sheet 230 and ventricular sheet 240 are substantially continuous but for the opening that leads to valve-receiving member 250. The second exception is that radiopaque markers 270 may be positioned directly on the atrial sheet 230 and ventricular sheet 240 at a "safe" area of passage. In Fig. 3, radiopaque markers 270 may be placed to help identify the location of the end(s) of the valve-receiving member 250, as well as the location of the end(s) of the shunt 280. The placement of radiopaque markers 270 may be similar in Fig. 11, even though no shunt is included. In other words, the radiopaque markers 270 may include a set of markers 270 that help identify an area between the valve-receiving member 250 and the inside of the waist 203 of the stent of anchor frame 201. If pacemaker leads need to be implanted after the prosthetic heart valve is implanted, one set of radiopaque markers 270 may be used to identify the area of the prosthetic heart valve where the lead may be passed through without causing damage to the leaflets within the valve-receiving member 250, without risking contact with the metal stent of the anchor frame 201, and without interfering with proper coaptation of the prosthetic leaflets after implantation of the leads. This trajectory of safe passage indicated by the relevant set of radiopaque markers 270 is indicated with broken line SP in Fig. 11.

In another embodiment, the designs of Fig. 3 and Fig. 11 may effectively be combined. In other words, a tube may be included between the atrial sheet 230 and the ventricular sheet 240, but the tube does not open to the atrial sheet 230 and ventricular sheet 240. In other words, upon implantation, although a shunt structure is in place, the shunt functionality is not in place because the atrial sheet 230 covers the first end of the tube, and the ventricular sheet 240 covers the second end of the tube. If it is desired to create a shunt at some point after implantation of the prosthetic heart valve, a physician may perform a later procedure to penetrate the atrial sheet 230 and ventricular sheet 240 where the tube is coupled, using the radiopaque markers 270 as a guide for the proper locations to penetrate. If desired, a stent or stent-like structure, similar to that shown in Fig. 6B, may be implanted into the shunt to help maintain the shunt open for blood to flow across the shunt. However, as with other embodiments, the implanted stent may include features to allow the newly formed shunt to close slowly over time. The same procedure may be followed if it is desired to create an open pathway for one or more pacemaker leads to be passed through the prosthetic heart valve during a pacemaker implantation procedure following implantation of the prosthetic heart valve.

Fig. 12 illustrates a variation of the design of Fig. 11. Fig. 12 is identical to the design of Fig. 11 with one exception. Instead of radiopaque markers 270 indicating a trajectory of safe passage, a guidewire GW may be pre-installed to the anchor frame 201. It should be noted that the embodiment of Fig. 12 may still include radiopaque markers 270 to identify the locations of the ends of the valve-receiving member 250. The guidewire GW may pass along substantially the same trajectory as the trajectory of safe passage of Fig. 11, or the position of the shunt 280 of Fig. 3 (even though no shunt is included in the illustrated embodiment of Fig. 12). In other words, the guidewire GW is positioned in the gap between the waist 203 and the valve-receiving member 250. Preferably, the guidewire GW includes a first end that extends beyond the ventricular sheet 240, and a second end that extends beyond the atrial sheet 230. Although not shown in Fig. 12, the guidewire GW preferably extends the entire length of the catheter used to implant the anchor frame 201, and the guidewire GW is preferably not permanently fixed to the anchor frame 201. If it is necessary to perform a pacemaker implantation procedure, the guidewire GW may be used as a rail to help ensure the safe passage of a secondary device through the prosthetic heart valve. If it is not necessary to perform a pacemaker implantation procedure, the guidewire GW may simply be removed from the patient. For example, if a pacemaker lead needs to be implanted, a catheter may be inserted into the patient with the catheter riding over the guidewire GW through the prosthetic heart valve, creating a desired pathway for the pacemaker lead. Similarly, if it is desired to create a shunt in a secondary procedure, the guidewire GW may be used to help position a secondary catheter in the desired position for the creation of a shunt. In some embodiments, a tube (covered by both the atrial sheet 230 and ventricular sheet 240) may be pre-assembled to the anchor frame 201 with the pre-assembled guidewire GW passing through the tube. In this embodiment, a stent implantation into the fabric tube to help create a shunt may be performed with the aid of guidewire GW for proper positioning. Fig. 13 illustrates the guidewire GW being used to guide a secondary catheter SC along the desired trajectory.

As noted above, a "docking" station-style anchor frame that is part of a two-stage implantation procedure is described herein to provide context for the shunts and pacemaker lead pathway features of this disclosure. But the invention is not limited to this type of prosthetic heart valve system and is instead equally applicable to single-stage prosthetic heart valve implants, including those with a two-stent configuration or a single-stent configuration.

Fig. 14 illustrates a prosthetic tricuspid valve 600 having features in common with the prosthetic heart valves described in U.S. Patent Application No. 63/341,702 filed May 13, 2022. Prosthetic heart valve 600 may take any of the forms shown and described in U.S. Patent Application No. 63/341,702, but one example is shown in Fig. 14. Prosthetic heart valve 600 includes a collapsible and expandable stent frame that includes an atrial disk 612, a ventricular disk 614, and a central portion 616. The central portion 616 may be cylindrical with an expanded diameter between about 25 mm and about 35 mm. A plurality of prosthetic leaflets 618 may be coupled to the central portion 616, for example by sutures. The prosthetic leaflets 618 are shown in a closed or coapted condition. The atrial disk 612 and ventricular disk 614 have expanded diameters that are larger than the central portion 616 and are configured to contact the atrial and ventricular sides of the native tricuspid valve annulus, for example sandwiching or clamping the annulus to help secure the prosthetic heart valve in place. An outer sealing fabric 620 may extend around the outer circumference of the prosthetic heart valve 600, extending from the atrial disk 612 to the ventricular disk 614. The outer sealing fabric 620 may be stretchable and permeable (*e.g*., if it is a knit fabric) or relatively non-stretchable and impermeable (*e.g*., if it is a tightly woven fabric). If the outer sealing fabric 620 is stretchable, it will not significantly interfere with the stent collapsing and expanding. If the outer sealing fabric 620 is non-stretchable, it may be pleated or otherwise shaped so as to not significantly interfere with the stent collapsing and expanding. The prosthetic heart valve 612 may also include an impermeably atrial sealing fabric 630 that generally follows the atrial disk 612 radially outward of the central portion 616. The atrial sealing fabric 630 and the prosthetic leaflets 618 may in combination provide for a complete seal or near-complete seal across the native valve annulus after implantation. There does not need to be any ventricular sealing fabric, and in fact, it may be preferable to exclude any such fabric so that retrograde blood flow during ventricular systole will tend to force the outer sealing fabric 620 into contact with the native valve annulus for enhanced sealing.

Still referring to Fig. 14, an opening 640 may be provided in the atrial sealing fabric 630 (which in some embodiments may actually be a tissue member instead of a synthetic fabric). This opening 640 will allow for blood to cross through the atrial sealing fabric 630, so that retrograde blood flow may occur across the prosthetic heart valve 600 even when the prosthetic leaflets 618 are closed. The size of the opening 640 may be designed to allow for the desired amount of regurgitation. As should be understood, this opening 640 may provide for similar shunt functionality as described above for other embodiments. Also, this opening 640 may provide a pathway for pacemaker leads. As with other embodiments, the opening 640 is positioned in a gap space between the inner valve assembly (which includes the central portion 616 of the stent in this embodiment) and the structure that will contact the native valve annulus (the outer sealing fabric 620 in this embodiment). It should be understood that the atrial disk 612 (as well as the rest of the stent) is preferably formed as a cellular structure (similar to that shown in Fig. 2) so that the metal of the stent does not hinder the shunt functionality or the ability for a pacemaker lead to pass through opening 640. As with other embodiments herein, the opening 640 may be sealed during a later intervention or may be designed to self-close over time. The opening 640 may be a "bare" opening or may be provided with a flap (not shown). For example, a fabric flap may be coupled to the atrial sealing fabric 630 directly adjacent the opening 640, so that the flap opens during ventricular systole (when pressure pushes the flap upwards, in the view of Fig. 14, to uncover the opening 640) and closes during ventricular diastole (when pressure pulls the flap down, in the view of Fig. 14, to cover the opening 640). This additional flap allows the shunt functionality to occur but may also help close the opening 640 permanently as ingrowth may occur at or on the flap to eventually lock the flap to permanently close the opening 640. This type of flap may be included in any of the shunts described in connection with other embodiments above. And it should be clear that the opening 640 may provide access for a delivery device and/or pacemaker lead, and radiopaque markers may be positioned adjacent to the opening 640 to assist with such delivery.

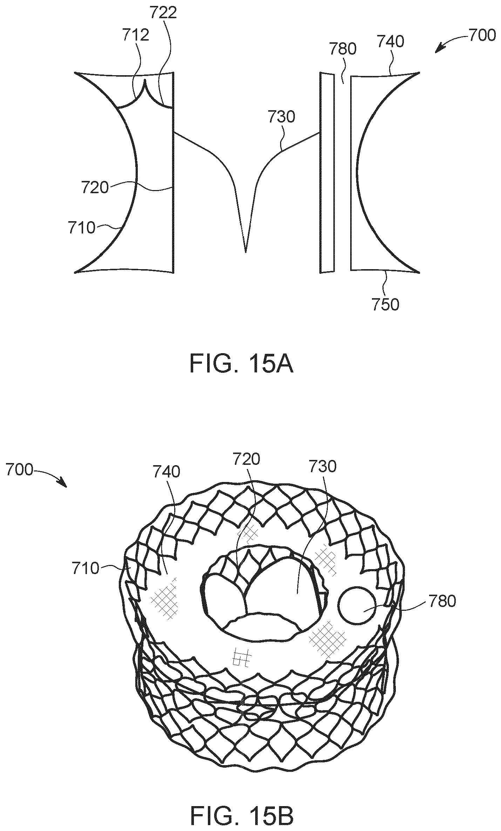

Figs. 15A-B show an example of a double-stented prosthetic heart valve 700 that incorporates a shunt 780. Prosthetic heart valve 700 may include an outer collapsible and expandable outer stent 710 (which may have an hourglass shape and be generally similar to that shown in Fig. 2) and a collapsible and expandable inner stent 720 (which may be generally cylindrical and formed of cells or a similar lattice structure). The outer stent 710 may be coupled to the inner stent 720 by any suitable mechanism. In this example, the outer stent 710 includes a plurality of inwardly extending arms 712, and the inner stent 720 includes a plurality of outwardly extending arms 722. These arms 712, 722 are positioned on the atrial side of the prosthetic heart valve 700 but could be positioned on the ventricular side. The arms 712, 722 may be coupled by any suitable mechanism, such as rivets or sutures. Preferably, the arms are provided at spaced distances around the circumference of the stent. As a result, the particular cross-section of Fig. 15A does not pass through a pair of arms on the right side of the prosthetic heart valve 700, but rather passes through an empty space between two circumferentially adjacent pairs of arms 712, 722. The prosthetic heart valve 700 may include sealing fabric 740 between the outer stent 710 and the inner stent 720 on the atrial side of the prosthetic heart valve and may include sealing fabric 750 between the outer stent 710 and the inner stent 720 on the ventricular side of the prosthetic heart valve 700. This configuration would result in blood only being able to pass through the prosthetic valve leaflets 730 when they are open, but for the shunt 780 included radially outward of the inner stent 720 and radially inward of the waist of the outer stent 710. The shunt 780 may take any of the forms described above, and the pacemaker lead safe passage features described above with other embodiments may be equally suited to the double stented prosthetic heart valve 700 of Figs. 15A-B. Thus, it should be clear that the shunt features and the pacemaker lead features described herein may be used with two-stage prosthetic heart valve implant systems as well as single-stage prosthetic heart valves, including those with a single stent or two stents.

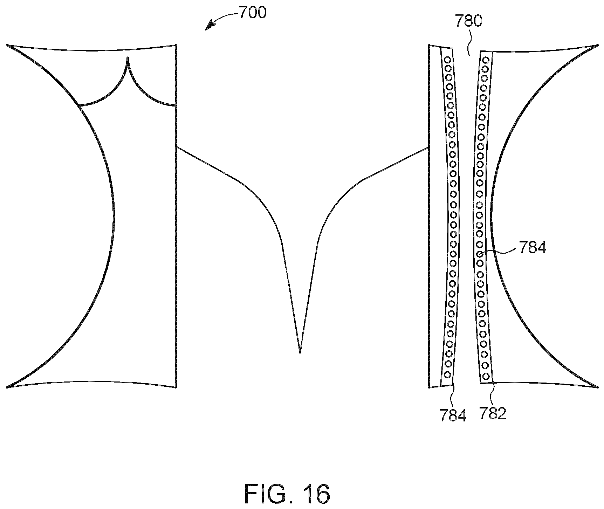

Fig. 16 illustrates prosthetic heart valve 700 with an additional feature. The shunt 780 of prosthetic heart valve 700 includes both an outer tube 782 and an inner tube 784. The outer tube 782 may be coaxial with or otherwise surround the inner tube 784. Trapped between the inner tube 784 and the outer tube 782 is a swellable material 786, such as microspheres formed of polyvinyl acid ("PVA"). One or both of the inner tube 784 and outer tube 782 is preferably formed of a semi-permeable material or other material that allows blood to infiltrate into the space between the two tubes, while preventing the swellable material 786 from crossing the tubes. Upon implantation, the swellable material 786 has a relatively small volume, and the shunt 780 has a relatively large cross-sectional area to allow blood to flow through the shunt 780. Over time, however, the swellable material 786 will begin to swell as a result of contact with blood. As the swellable material 786 swells, it pushes on the inner tube 784 to reduce the cross-sectional area of the shunt 780, slowly closing the shunt over time, until preferably the swellable material 786 swells enough to fully close the shunt 780. It should be understood that this closing mechanism may be provided for any of the other shunts described herein.

## Claims

1. A prosthetic heart valve system for replacing a native right atrioventricular valve, the system comprising:
a collapsible and expandable anchor frame (101, 201) including:
a central portion (103, 203, 616), and an atrial portion (102, 202, 612) and ventricular portion (104, 204, 614) each flared radially outwardly from the central portion, the atrial and ventricular portions sized to sandwich an annulus of the right atrioventricular valve therebetween;
an atrial sheet (230, 630, 740) coupled to the atrial portion of the anchor frame and extending radially inwardly to a central aperture in the atrial sheet, the atrial sheet being formed of a material that is substantially impermeable to blood;
a ventricular sheet (240, 750) coupled to the ventricular portion of the anchor frame and extending radially inwardly to a central aperture in the ventricular sheet; and
a generally cylindrical leaflet support structure, an inflow end of the leaflet support structure coupled to the atrial sheet;
**characterised in that** the atrial sheet defines an opening positioned radially outside of the leaflet support structure, the ventricular sheet defines an opening positioned radially outside of the leaflet support structure, a tube extending from the opening in the ventricular sheet to the opening in the atrial sheet to form a shunt (280, 480, 580, 780) so that, upon implantation of the anchor frame into the native right atrioventricular valve, blood flowing around an exterior of the leaflet support structure can pass through the openings and through the shunt in a retrograde direction and in antegrade direction,
wherein the tube forming the shunt is either cylindrical, or bean-shaped in transverse cross-section,
wherein a closure member (484, 584) is coupled to the shunt (280), the closure member including a bioabsorbable member (488, 588) maintaining the closure member in an open condition in which blood is free to flow through the shunt.

2. The prosthetic heart valve system of claim 1, wherein the leaflet support structure is the central portion of the anchor frame (616), and an outer sealing fabric (620) extends between the atrial and ventricular portions (612, 614) of the anchor frame.

3. The prosthetic heart valve system of claim 1, wherein the anchor frame is an outer stent (710), and the leaflet support structure is an inner stent (720).

4. The prosthetic heart valve system of claim 1, wherein the anchor frame is an outer stent (101), and the leaflet support structure is a fabric tube (250).

5. The prosthetic heart valve system of claim 4, further comprising a collapsible and expandable prosthetic heart valve (300) including a stent (320) and a plurality of prosthetic leaflets (340), the prosthetic heart valve configured to be expanded into and received within the fabric tube (250) of the anchor frame.

6. The prosthetic heart valve system of claim 1, wherein the closure member (484) is formed of a shape-memory material, the closure member including two apices and two "C"-shaped sides, the two "C"-shaped sides nesting with each other in the absence of applied forces, the bioabsorbable member being a wire connected to the two apices of the closure member, the wire maintaining the closure member in an open condition in which the two "C"-shaped sides form a generally circular passageway, the tube of the shunt passing through the generally circular passageway.

7. The prosthetic heart valve system of claim 1, wherein the closure member (584) is formed of a shape-memory material, the closure member having a closed condition in the absence of applied forces, the closure member forming a spiral shape with an interior diameter when in the closed condition, and wherein in the open condition of the closure member, two ends of the closure member overlap, the two ends being coupled together by the bioabsorbable member (588, 588') so that an interior diameter of the closure member in the open condition is larger than the interior diameter of the closure member in the closed condition.

8. The prosthetic heart valve system of claim 7, wherein the bioabsorbable member is either a sheath (588') that receives the two ends of the closure member (584) within the sheath, or a wire (588) that wraps around the two ends of the closure member.

9. The prosthetic heart valve system of any of the preceding claims, further comprising a radiopaque marker (270) coupled to the atrial sheet (230, 630, 740) adjacent to the opening.

10. The prosthetic heart valve system of any of claims 1-4, further comprising a plurality of prosthetic leaflets (618, 730) directly coupled to the leaflet support structure, the prosthetic leaflets forming a valve that allows blood to flow from the inflow end of the leaflet support structure to the outflow end of the leaflet support structure, but generally blocks blood from flowing from the outflow end of the leaflet support structure to the inflow end of the leaflet support structure.

## Patentansprüche

1. Ein Herzklappenprothesensystem zum Ersatz einer natürlichen rechten atrioventrikulären Klappe, wobei das System einen kollabierbaren und expandierbaren Verankerungsrahmen (101, 201) aufweist,
mit einem mittleren Abschnitt (103, 203, 616) und mit einem Vorhofabschnitt (102, 202, 612) und einem Ventrikelabschnitt (104, 204, 614), die jeweils radial nach außen vom mittleren Abschnitt aus aufgeweitet sind, wobei der Vorhof- und der Ventrikelabschnitt so dimensioniert sind, dass sie einen Annulus der rechten atrioventrikulären Klappe zwischen sich einschließen;
mit einem Vorhoftuch (230, 630, 740), das mit dem Vorhofabschnitt des Verankerungsrahmens verbunden ist und sich radial nach innen zu einer zentralen Öffnung in dem Vorhoftuch erstreckt, wobei das Vorhoftuch aus einem Material gebildet ist, das für Blut im Wesentlichen undurchlässig ist;
mit einem Ventrikeltuch (240, 750), das mit dem Ventrikelabschnitt des Verankerungsrahmens verbunden ist und sich radial nach innen zu einer zentralen Öffnung in dem Ventrikeltuch erstreckt; und
mit einer im Allgemeinen zylindrische Klappenstützstruktur, wobei ein Einströmende der Klappenstützstruktur mit dem Vorhoftuch verbunden ist;
**dadurch gekennzeichnet, dass** das Vorhoftuch eine Öffnung definiert, die radial außerhalb der Klappenstützstruktur positioniert ist, das Ventrikeltuch eine Öffnung definiert, die radial außerhalb der Klappenstützstruktur positioniert ist, sich ein Schlauch von der Öffnung in dem Ventrikeltuch zur Öffnung in dem Vorhoftuch erstreckt, um einen Shunt (280, 480, 580, 780) zu bilden, so dass bei Implantation des Verankerungsrahmens in die natürliche rechte atrioventrikuläre Klappe das außerhalb der Klappenstützstruktur fließende Blut durch die Öffnungen und den Shunt in retrograder Richtung und in antegrader Richtung fließen kann,
wobei der den Shunt bildende Schlauch entweder zylindrisch oder im Querschnitt bohnenförmig ist, und
wobei ein Abschlusselement (484, 584) mit dem Shunt (280) verbunden ist, wobei das Abschlusselement ein bioabsorbierbares Element (488, 588) aufweist, das das Abschlusselement in einem offenen Zustand hält, in welchem Blut ungehindert durch den Shunt fließen kann.

2. Das Herzklappenprothesensystem nach Anspruch 1, wobei die Klappenstützstruktur der mittlere Abschnitt des Verankerungsrahmens (616) ist und wobei sich ein äußeres Dichtungsgewebe (620) zwischen dem Vorhofabschnitt und dem Ventrikelabschnitt (612, 614) des Verankerungsrahmens erstreckt.

3. Das Herzklappenprothesensystem nach Anspruch 1, wobei der Verankerungsrahmen ein äußerer Stent (710) ist und die Klappenstützstruktur ein innerer Stent (720) ist.

4. Das Herzklappenprothesensystem nach Anspruch 1, wobei der Verankerungsrahmen ein äußerer Stent (101) ist und die Klappenstützstruktur ein Gewebeschlauch (250) ist.

5. Das Herzklappenprothesensystem nach Anspruch 4, das ferner eine kollabierbare und expandierbare Herzklappenprothese (300) aufweist, die einen Stent (320) und eine Mehrzahl von Prothesenklappen (340) aufweist, wobei die Herzklappenprothese so konfiguriert ist, dass sie in den Gewebeschlauch (250) des Verankerungsrahmens expandiert und darin aufgenommen werden kann.

6. Das Herzklappenprothesensystem nach Anspruch 1, wobei das Abschlusselement (484) aus einem Formgedächtnismaterial gebildet ist, wobei das Abschlusselement zwei Spitzen und zwei C-förmige Seiten aufweist, wobei die beiden C-förmigen Seiten bei Abwesenheit von aufgebrachten Kräften ineinander verschachtelt sind, wobei das bioabsorbierbare Element ein Draht ist, der mit den beiden Spitzen des Abschlusselements verbunden ist, wobei der Draht das Abschlusselement in einem offenen Zustand hält, in dem die beiden C-förmigen Seiten einen im Allgemeinen kreisförmigen Durchgang bilden, wobei der Schlauch des Shunts durch den im Allgemeinen kreisförmigen Durchgang verläuft.

7. Das Herzklappenprothesensystem nach Anspruch 1, wobei das Abschlusselement (584) aus einem Formgedächtnismaterial gebildet ist, wobei das Abschlusselement in Abwesenheit von aufgebrachten Kräften einen geschlossenen Zustand aufweist, wobei das Abschlusselement im geschlossenen Zustand eine Spiralform mit einem Innendurchmesser bildet, und wobei im offenen Zustand des Abschlusselements sich die beiden Enden des Abschlusselements überlappen, wobei die beiden Enden durch das bioabsorbierbare Element (588, 588') miteinander verbunden sind, so dass der Innendurchmesser des Abschlusselements im geöffneten Zustand größer ist als der Innendurchmesser des Abschlusselements im geschlossenen Zustand.

8. Das Herzklappenprothesensystem nach Anspruch 7, wobei das bioabsorbierbare Element entweder eine Hülse (588') ist, die die beiden Enden des Abschlusselements (584) innerhalb der Hülse aufnimmt, oder ein Draht (588), der die beiden Enden des Abschlusselements umschließt.

9. Das Herzklappenprothesensystem nach einem der vorstehenden Ansprüche, das ferner eine röntgendichte Markierung (270) aufweist, die an dem Vorhoftuch (230, 630, 740) benachbart zur Öffnung angebracht ist.

10. Das Herzklappenprothesensystem nach einem der Ansprüche 1 bis 4, das ferner eine Mehrzahl von Prothesenklappen (618, 730) aufweist, die direkt mit der Klappenträgerstruktur verbunden sind, wobei die Prothesenklappen ein Klappenventil bilden, das den Blutfluss vom Einströmende der Klappenträgerstruktur zum Ausströmende der Klappenträgerstruktur ermöglicht, aber im Allgemeinen den Blutfluss vom Ausströmende der Klappenträgerstruktur zum Einströmende der Klappenträgerstruktur blockiert.

## Revendications

1. Un système de valve cardiaque prothétique destiné à remplacer une valve auriculo-ventriculaire droite native, le système comprenant :
un cadre d'ancrage repliable et extensible (101, 201) comprenant :
une partie centrale (103, 203, 616), et une partie auriculaire (102, 202, 612) et une partie ventriculaire (104, 204, 614) chacune s'évasant radialement vers l'extérieur à partir de la partie centrale, les parties auriculaire et ventriculaire étant dimensionnées pour prendre en sandwich un anneau de la valve auriculo-ventriculaire droite entre elles;
une feuille auriculaire (230, 630, 740) couplée à la partie auriculaire du cadre d'ancrage et s'étendant radialement vers l'intérieur jusqu'à une ouverture centrale dans la feuille auriculaire, la feuille auriculaire étant formée d'un matériau qui est sensiblement imperméable au sang;
une feuille ventriculaire (240, 750) couplée à la partie ventriculaire du cadre d'ancrage et s'étendant radialement vers l'intérieur jusqu'à une ouverture centrale dans la feuille ventriculaire; et
une structure de support de feuillet de valvule généralement cylindrique, une extrémité d'entrée de la structure de support de feuillet de valvule est couplée à la feuille auriculaire;
**caractérisé en ce que** la feuille auriculaire définit une ouverture positionnée radialement à l'extérieur de la structure de support de feuillet de valvule, la feuille ventriculaire définit une ouverture positionnée radialement à l'extérieur de la structure de support de feuillet de valvule, un tube s'étendant depuis l'ouverture dans la feuille ventriculaire jusqu'à l'ouverture dans la feuille auriculaire pour former un shunt (280, 480, 580, 780) de sorte que, lors de l'implantation du cadre d'ancrage dans la valve auriculo-ventriculaire native droite, le sang s'écoulant autour de l'extérieur de la structure de support de feuillet de valvule puisse passer à travers les ouvertures et à travers le shunt dans une direction rétrograde et dans une direction antérograde,
le tube formant le shunt étant soit cylindrique, soit en forme de haricot en coupe transversale,
dans lequel un élément de terminaison (484, 584) est couplé à le shunt (280), l'élément de terminaison comprenant un élément bioabsorbable (488, 588) maintenant l'élément de terminaison dans un état ouvert dans lequel le sang peut s'écouler librement à travers le shunt.

2. Le système de valve cardiaque prothétique selon la revendication 1, dans lequel la structure de support de feuillet de valvule est la partie centrale du cadre d'ancrage (616), et un tissu d'étanchéité extérieur (620) s'étend entre les parties auriculaire et ventriculaire (612, 614) du cadre d'ancrage.

3. Le système de valve cardiaque prothétique selon la revendication 1, dans lequel le cadre d'ancrage est un stent externe (710) et la structure de support de feuillet de valvule est un stent interne (720).

4. Le système de valve cardiaque prothétique selon la revendication 1, dans lequel le cadre d'ancrage est un stent externe (101) et la structure de support de feuillet de valvule est un tube en tissu (250).

5. Le système de valve cardiaque prothétique selon la revendication 4, comprenant en outre une valve cardiaque prothétique repliable et expansible (300) comprenant un stent (320) et une pluralité de feuillet de valvule prothétiques (340), la valve cardiaque prothétique étant configurée pour être expansée et reçue à l'intérieur du tube en tissu (250) du cadre d'ancrage.

6. Le système de valve cardiaque prothétique selon la revendication 1, dans lequel l'élément de terminaison (484) est formé d'un matériau à mémoire de forme, l'élément de terminaison comprenant deux sommets et deux côtés en forme de « C », les deux côtés en forme de « C » s'emboîtant l'un dans l'autre en l'absence de forces appliquées, l'élément bioabsorbable étant un fil relié aux deux sommets de l'élément de terminaison, le fil maintenant l'élément de terminaison dans un état ouvert dans lequel les deux côtés en forme de « C » forment un passage généralement circulaire, le tube de le shunt passant à travers le passage généralement circulaire.

7. Le système de valve cardiaque prothétique selon la revendication 1, dans lequel l'élément de terminaison (584) est formé d'un matériau à mémoire de forme, l'élément de terminaison ayant une position fermée en l'absence de forces appliquées, l'élément de terminaison formant une forme en spirale avec un diamètre intérieur lorsqu'il est en position fermée, et dans lequel, en position ouverte de l'élément de terminaison, les deux extrémités de l'élément de terminaison se chevauchent, les deux extrémités étant couplées ensemble par l'élément bioabsorbable (588, 588') de sorte que le diamètre intérieur de l'élément de terminaison dans la position ouverte est plus grand que le diamètre intérieur de l'élément de terminaison dans la position fermée.

8. Le système de valve cardiaque prothétique selon la revendication 7, dans lequel l'élément bioabsorbable est soit une gaine (588') qui reçoit les deux extrémités de l'élément de terminaison (584) à l'intérieur de la gaine, soit un fil (588) qui s'enroule autour des deux extrémités de l'élément de terminaison.

9. Le système de valve cardiaque prothétique selon l'une quelconque des revendications précédentes, comprenant en outre un marqueur radio-opaque (270) couplé à la feuille auriculaire (230, 630, 740) adjacente à l'ouverture.

10. Le système de valve cardiaque prothétique de l'une quelconque des revendications 1 à 4, comprenant en outre une pluralité de feuillet de valvule prothétiques (618, 730) directement couplés à la structure de support de feuillet de valvule, les feuillets de valvule prothétiques formant une valve qui permet au sang de s'écouler de l'extrémité d'entrée de la structure de support de feuillet de valvule vers l'extrémité de sortie de la structure de support de feuillet de valvule, mais qui empêche généralement le sang de s'écouler de l'extrémité de sortie de la structure de support de feuillet de valvule vers l'extrémité d'entrée de la structure de support de feuillet de valvule.
